(19) Europäisches Patentamt
European Patent Office
Office européen des brevets

(11) Publication number: **0 184 787 B1**

# (12) EUROPEAN PATENT SPECIFICATION

(45) Date of publication of patent specification: **03.04.91** (51) Int. Cl.⁵: **G01N 33/72**

(21) Application number: **85115530.9**

(22) Date of filing: **06.12.85**

## (54) Cyanide-free hemoglobin reagent.

(30) Priority: **06.12.84 US 679149**

(43) Date of publication of application:
**18.06.86 Bulletin 86/25**

(45) Publication of the grant of the patent:
**03.04.91 Bulletin 91/14**

(84) Designated Contracting States:
**DE FR GB IT**

(56) References cited:
**EP-A- 0 003 340**
**GB-A- 2 147 999**
**US-A- 3 677 707**
**US-A- 4 372 747**

**CHEMICAL ABSTRACT, vol. 89, no. 9, 28th August 1978, page 115, column 2, abstract no. 71645n, Columbus, Ohio, US; L. BOLIS et al.: "Action of some anionic and non-ionic detergents on human erythrocyte membranes";**

**PATENT ABSTRACTS OF JAPAN, vol. 5, no. 201 (94)[873], 19th December 1981; & JP - A - 56 120 951 (WAKO JUNYAKU KOGYO K.K.) 22-09-1981**

(73) Proprietor: **TECHNICON INSTRUMENTS CORPORATION(a Delaware corporation)**
**511 Benedict Avenue**
**Tarrytown, New York 10591-6097(US)**

(72) Inventor: **Benezra, Jeffrey**
**140-18 Debs Place**
**Bronx New York(US)**
Inventor: **Malin, Michael Joel**
**14 Terrace Circle**
**Park Ridge New Jersey(US)**

(74) Representative: **Patentanwälte Grünecker, Kinkeldey, Stockmair & Partner**
**Maximilianstrasse 58**
**W-8000 München 22(DE)**

EP 0 184 787 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a cyanide-free aqueous solution and a method for determining the total concentration of hemoglobins (Hb) in whole blood or in specifically prepared control and calibrator materials derived from whole blood.

The determination of the total concentration of hemoglobins is indicative of the oxygen-carrying capacity of whole blood. The reference, and most commonly used method, for determination of total hemoglobin is the cyanmethemoglobin method. In this method, ferrous ion (Fe (II)), of heme in hemoglobin, oxyhemoglobin and carboxyhemoglobin of the red blood cell is oxidized to the ferric state (Fe (III)) by ferricyanide to form methemoglobin. Methemoglobin is then combined with ionized cyanide to produce cyanmethemoglobin, which is measured photometrically at 540 nm. For further background, reference is made to Henry, et al. (Eds.) Clinical Chemistry, Harper & Row, Hagerstown, MD (1974) at page 1131 et seq; Drabkin, J. Biol. Chem., 112:51 (1935); and Van Kampen and Ziljstrs, Advances in Clinical Chemistry, 8 :141-187.

US-A-3,874,852 discloses a reagent for hemoglobin determination, which includes ionic cyanide in an alkaline aqueous solution having a pH of 9. In such reagents, ferricyanide is not present. Rather, the heme is oxidized to the ferric state by atmospheric oxygen. The ferric hemoglobin species then binds to the cyanide ions to produce a chromogen which is measured to quantify the hemoglobin.

US-A-4,286,963 discloses a reagent for determining both lymphoid and myeloid leukocytes and hemoglobin in whole blood. The reagent includes a surface-active quaternary ammonium salt, a phenyl or phenoxy alkanol, and a polyalcohol in an acidic buffer (pH 3.5-5.0) and does not contain ionic cyanide. The suggestion is made that the lack of ionic cyanide is undesirable and results in instability of the chromogen formed by reaction with the hemoglobin. Said patent suggests that, if ionic cyanide is present in the reagent, the reagent should be at an alkaline pH for inclusion of buffered cyanide, so as to obtain a satisfactory hemoglobin derivative.

Clin. Biochem. 15 ,83 (19821, teaches the use of a reagent for hemoglobin determination which comprises sodium dodecyl sulfate or, equivalently, sodium lauryl sulfate (SLS), an anionic surfactant, and Triton® X-100, a nonionic surfactant, in a neutral buffer (pH 7.2). The red blood cells are lysed by the SLS. The presence of Triton X-100 prevents SLS from precipitating at temperatures below 5 °C. The reaction is completed within 5 to 10 min and produces a green chromogen having absorption maxima at 539 and 572 nm, the depth of color being indicative of the hemoglobin content.

Clin. Chem. Acta, 136 (1984) also disclose a method for determining total hemoglobin concentration, utilizing a reagent which consists of a nonionic surfactant, such as Triton X-100, dissolved in 0.1 N NaOH, a strongly alkaline medium. The reaction is completed within 1 to 2 min and a green chromogen is formed having an absorption maximum at 575 nm and a shoulder at 600 nm. Said document specifically states that the method does not function if nonionic surfactants are replaced by either cationic or anionic surfactants.

The high throughput of current automated hematologic systems requires the use of methods with rapid turnover, e.g., completion time less than 30 s. In the case of hemoglobin determinations, such rapid turnover has only been achieved in the prior art by the use of cyanide-containing reagents of high pH and high cyanide levels. Hence, these reagents are highly toxic and also unstable, since ionic cyanide undergoes base-promoted hydrolysis to form formamide and formate. Consequently, additional cyanide must be introduced in the reagent to compensate for such hydrolytic degradation.

The prior art methods described above are characterized by the use of ionic cyanide, ferricyanide or nonionic surfactants. One of the disadvantages of the prior art is that the completion time to form the chromogen is substantially in excess of 30 s.

Also, because of environmental reasons, the presence of ionic cyanide in any type of reagent used for analytical purposes is highly undesirable. The disposal of the reaction mixture, or effluent, may require special treatment to reduce the concentration of the ionic cyanide within prescribed limits. Otherwise, if in excess of such limits, disposal of the effluent will require special measures, which are costly. Obviously, effluent disposal is very much simplified if no ionic cyanide, or other toxic material, is present in the effluent.

EP-A-3340 discloses a method and a reagent for determining the hemoglobin concentration in blood which comprises a water-soluble non-ionic detergent.

Chemical Abstracts, Vol. 89, No. 9, 28.8.1978, page 115, col. 2, No. 71645 n compares anionic and non-ionic detergents on human erythrocyte membranes.

GB-A-2147999 discloses a lysing agent comprising a mixture of an aqueous solution of at least two quaternary ammonium salts and an alkali metal cyanide.

It is the object of the present invention to provide a reagent and a method for determining the

concentration of hemoglobins in blood which has a rapid turnover and which does not require the use of cyanide.

Said object is achieved by a cyanide-free aqueous solution for determining the total hemoglobins concentration in a blood sample comprising an ionic surfactant having a pH of at least 11.3, said surfactant containing an alkyl hydrophobic member containing up to 30 carbon atoms including a principal chain containing from 10 to 20 carbon atoms and a polar group which is zwitterionic, cationic or anionic.

Furthermore, a method for determining the total concentration of hemoglobins in a blood sample is provided which comprises:

(a) treating red blood cells in a blood sample with the above aqueous solution wherein an oxidized heme product is formed and

(b) measuring the absorbance of said oxidized heme product and calculating the hemoglobins concentration therefrom.

The solution of the present invention is free of ionic cyanide and capable of performing hemoglobin determinations faster than prior methods, e.g., less than 30 s, so as to be particularly suited for present-day automated instrumentation. The assay method and reagent composition of the invention are particularly faster than prior art methods in analyzing blood samples containing various forms of hemoglobins, e.g., carboxyhemoglobin, such as in the blood of patients exposed to high levels of carbon monoxide, and are free of the toxicity associated with the presence of ionic cyanide. Sample fluids on which such determinations are performed include fresh whole blood and also specially prepared controls and calibrators derived from whole blood used to calibrate hematology analyzers.

The solution of the present invention comprises an ionic surfactant at a pH sufficiently basic for the denaturation of hemoglobin and oxidation of the heme iron in said hemoglobin to form an oxidized heme product, the absorption maxima of which is in the visible range.

The reaction product has a distinct green color and is quite stable. The visible absorption spectrum of the reaction product was found to superimpose with published spectra of hemin chloride dissolved in a solvent containing a surfactant, such as hexadecyltrimethylammonium bromide or, equivalently, cetyltrimethyl ammonium bromide (CTAB) at an alkaline PH. It was identified as a ferric protoporphyrin IX derivative in which hydroxide ions are the axial ligands of the centrally coordinated ferric ion (Simplicio and Schwenzer, Biochem. 12 (1973) 1923). The same reaction product, is formed from either native hemoglobin in whole blood, solutions of methemoglobin or hemin chloride.

In the reaction of native hemoglobin in whole blood with the novel reagent composition of the present invention, surfactant rapidly lyses the red blood, thereby releasing hemoglobin, and efficiently and completely emulsifying the cellular debris and the high lipid content of lipidic plasma of certain blood sample. Since the reagent completely disperses all sources of turbidity arising from the sample, neither filtration nor centrifugation is required prior to optical measurement.

The hemoglobin is then denatured, with the base and surfactant playing distinct roles. The base provides an alkaline pH which denatures the quaternary structure of the hemoglobin by essentially eliminating electrostatic ("salt-bridge") interactions. The surfactant denatures the hemoglobin by interacting with the interior hydrophobic portion of the protein to expose the heme. Denaturation caused by base and surfactant, in combination, occurs at a faster rate than achievable by either component alone. The denaturation releases the hemes (each hemoglobin possess four hemes) from their non-covalent interaction with the hydrophobic interior of globin. The hemes are extracted into the micelles of the surfactant, where contact with atmospheric oxygen causes rapid oxidation of the heme iron to the ferric state (Fe III). Subsequent reaction of the ferric heme with hydroxide ions leads to the reaction product, in which hydroxide ions are axial ligands of the resulting ferric ion. The resulting reaction product is very stable, as evidenced by retention of its absorption spectrum over a period of weeks when the reacted sample is stored at room temperature.

In order to be most effective, the present composition must increase the alkalinity of the blood sample. The ionic surfactant of the composition may serve as a base. Alternatively, a strong base independent of the surfactant may be included in the composition to impart the required pH of at least 11.3 for the reaction. It is preferred that the pH be greater than 13.7.

In general, the ionic surfactants of the present composition must contain a long alkyl hydrophobic chain and one polar head group. The alkyl group contains from 10 to 20 carbon atoms on the principal chain and up to a total of 30 carbon atoms. It is preferred that the alkyl group contains from 12 to 18 carbon atoms on the principal chain and up to a total of 24 carbon atoms. It is especially preferred that the alkyl chain contains 12 to 18 carbon atoms and is unbranched. Examples of $C_{12}$-$C_{18}$ alkyl group include lauryl (or dodecyl), myristyl (or tetradecyl), stearyl (or octadecyl) or cetyl (hexadecyl).

The long alkyl hydrocarbyl chain of the surfactant is attached to a head group. The head group may be

cationic, anionic, or zwitterionic. The long alkyl hydrocarbyl chain is attached to the head group through a covalent bond. There may be more than one alkyl group attached to a head group. These alkyl groups may be the same or different. They may be straight chained or branched and contain from 1 to 20 carbon atoms. Preferably, they are lower alkyl groups containing from 1 to 6 carbon atoms. Such groups include methyl, ethyl, propyl, isopropyl, butyl, isobutyl, amyl, isoamyl and hexyl. Especially preferred alkyl groups are methyl.

The compounds used in present invention may contain one or more asymmetric carbon atoms and may exist in racemic and optically active forms. All of these compounds are contemplated to be within the scope of the present invention.

There are four different types of ionic surfactants. As suggested above, one type of surfactant may serve as a base and can impart the required pH independently of any additional base which may be present. Surfactants suitable to impart the required pH include alkyl ammonium hydroxides of the formula:

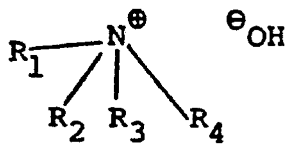

wherein

$R_1$ is $C_{12}$-$C_{18}$ alkyl;

$R_2$, $R_3$ and $R_4$ may be the same or different and are each independently alkyl.

It is preferred that $R_2$, $R_3$ and $R_4$ be lower alkyl. Especially preferred ionic surfactants are the $C_{12}$-$C_{18}$ alkyl trimethylammonium hydroxides. Examples of such ionic surfactants include octadecyltrimethylammonium hydroxide or equivalently, stearyltrimethylammonium hydroxide; dodecyltrimethylammonium hydroxide or equivalently, lauryl trimethylammonium hydroxide; tetradecyltrimethylammonium hydroxide, or equivalently, myristyltrimethylammonium hydroxide. These ionic surfactants belong to the class of cationic surfactants. However, as shown below, not all cationic surfactants possess this quality.

Many ionic surfactants require a strong base independent of the surfactant to be included in the composition in order to impart the required pH for the efficacy of the present invention. These ionic surfactants include cationic surfactants, zwitterionic surfactants and anionic surfactants.

A second group of surfactants are those cationic surfactants which are not basic enough to impart the required pH by themselves. Cationic surfactants within this class include quaternary ammonium salts, especially the halides. More specifically, these cationic surfactants include $C_{12}$-$C_{18}$ alkylammonium salts of the formula:

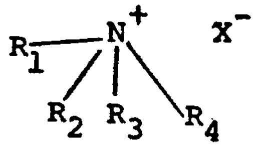

wherein

$R_1$ is $C_{12}$-$C_{18}$ alkyl;

$R_2$, $R_3$ and $R_4$ are independently hydrogen, or alkyl; and

X is halide.

It is preferred that $R_2$, $R_3$ and $R_4$ are lower alkyl. Especially preferred cationic surfactants are the $C_{12}$-$C_{18}$ alkyl trimethylammonium halides. Examples include stearyltrimethylammonium halide, lauryltrimethylammonium halide, myristyltrimethylammonium halide and cetyltrimethylammonium halide. Preferred halides are chlorides or bromides.

Other alkylammonium salts are also encompassed within the present invention. These counterions include those bases which act as "spectators", are non-toxic, and do not interfere with the visible absorption of the product, e.g., sulfates, nitrates, oxalates or acetates.

Another class of surfactants which require an independent base to impart the required pH are zwitterionic surfactants. Surfactants within this class include $C_{12}$-$C_{18}$ alkylamine N-oxides of the formula:

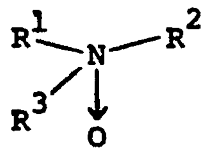

wherein

$R_1$ is $C_{12}$-$C_{18}$ alkyl;

$R_2$ and $R_3$ may be the same or different and are each independently alkyl.

Preferred alkyl groups are lower alkyl. Especially preferred zwitterionic surfactants include the N,N-dimethyl-$C_{12}$-$C_{18}$ alkyl amine N-oxide. Examples of zwitterionic surfactants include N,N-dimethyllauryl amine N-oxide, N,N-dimethylcetylamine N-oxide and N,N-dimethylstearylamine N-oxide.

The fourth class of ionic surfactants are the anionic surfactants. This class also works most effectively when it is in combination with an independent base to impart the efficacious pH. Surfactants within this class include alkali and alkaline earth metal salts of $C_{12}$-$C_{18}$ alkyl sulfates. Examples of anionic surfactants include sodium lauryl sulfate, lithium lauryl sulfate and sodium myristyl sulfate. Examples of strong bases which are suitable to impart the required pH include alkali metal hydroxides, such as sodium hydroxide and potassium hydroxide. Another example is a tetralkylammonium hydroxide, where the alkyl group can contain 1-4 carbon atoms, such as tetrabutylammonium hydroxide.

The preferred range of the surfactant concentration is between 2.0-5.0 g/dl. Also, the preferred range of the base concentration, where used independently of the surfactant to establish the required pH, is between 0.4-0.6 N.

Unlike prior art methods, reliable hemoglobin determinations are made by observing the absorbence of the reaction mixture within about 30 s and without the risk accompanying the presence of ionic cyanide.

The ionic surfactants used in this invention are either readily available or can be prepared from known compounds or readily available intermediates by art-recognized techniques, such as substitution or conversion. For example, alkyl sulfonates can be prepared by treating the corresponding alkyl sulfonic acid with a strong base, e.g., sodium hydroxide, lithium hydroxide or potassium hydroxide.

The alkyl sulfonic acid is prepared, in turn, by treating the corresponding alcohol or halide with sulfur trioxide, sulfuric acid or halosulfonic acid, e.g., chlorosulfonic acid, by techiques well-known in the art. A coupling agent, such as dicyclohexylcarbodimides, may also be present.

The ammonium ionic surfactants can also be prepared by art recognized techniques. For example, the $C_{12}$-$C_{18}$ alkyl ammonium halide can be prepared by treating the corresponding alkyl halides with excess ammonia and isolating the salt thereof. The secondary alkyl ammonium salt can be prepared by reacting an excess of a primary amine with an alkyl halide and isolating the salt thereof. The primary amine can be formed by various methods, e.g., treating the primary alkyl ammonium halide formed above with base, e.g., $NH_3$ or hydroxide. Alternatively, the primary amine can be prepared by other well known organic methods known in the art, such as Hofmann degradation of the corresponding amides, reduction of the corresponding nitriles, and reductive amination of the corresponding aldehyde or ketone with ammonia. Similarly tertiary alkyl ammonium halides can be prepared by reacting an excess of secondary amine with alkyl halide. The secondary amine can be prepared by treating the secondary alkyl ammonium salt with base, such as hydroxide or ammonia. Other methods in the art for forming the secondary amine include reductive amination of an aldehyde or ketone with a primary amine. The quaternary alkyl ammonium salt can be prepared by reacting the tertiary alkyl amine with alkyl halide. The tertiary alkylamine can be prepared in a similar manner as described above. For example, it can be prepared from the tertiary alkyl ammonium salt followed by reaction with a base, e.g., hydroxide or ammonia, or by reductive amination of a secondary amine with an aldehyde or ketone. Alternatively, if $R_2$, $R_3$ and $R_4$ are the same, the quaternary alkyl ammonium salt can be prepared by exhaustive alkylation of the $C_{12}$-$C_{18}$ alkyl amines with an excess of alkyl halide.

The quaternary alkyl ammonium hydroxide can be prepared by techniques known in the art. For example, the quaternary ammonium halide is treated with silver oxide, which precipitates out the silver halide. When the mixture is filtered and the filtrate evaporated off, the quaternary ammonium hydroxide is formed.

The amine N-oxides can also be prepared by well recognized techniques known in the art. For example, the appropriate tertiary amine is converted to amine oxides with standard oxidizing agents, such as hydrogen peroxide, peracids, and Caro's acid ($H$-$O$-$O$-$SO_3H$).

The method of the present invention generally involves detection of a specific product derived by the reaction of the novel reagent composition of the invention with naturally occurring hemoglobin species present in whole blood sample (or control or calibrator material). Naturally occurring hemoglobin species are deoxyhemoglobin, oxyhemoglobin, methemoglobin, fetalhemoglobin, carboxyhemoglobin and sickle cell hemoglobin. The blood sample is diluted about 250 fold with the reagent composition and the resulting reaction product having a reproducible absroption spectrum with a maximum absorption at 570 nm and a shoulder at 595 nm. The present composition is capable of determining the concentration of hemoglobin in whatever form it is present.

One possible mechanism of the reaction is supported by the following equations:

$$(1)\quad \text{WB} + \text{DMLAO} + \text{NaOH} \longrightarrow \text{GP-1}\ (\lambda\ \text{max}\ 570,\ 595\text{sh nm})$$

$$(2)\quad \text{WB} + \text{SLS} + \text{NaOH} \longrightarrow \text{GP-2}\ (\lambda\ \text{max}\ 495,\ 525\text{sh},\ 603\ \text{nm})$$

$$(3)\quad \text{WB} + \text{CTAB} + \text{NaOH} \longrightarrow \text{GP-3}\ (\lambda\ \text{max}\ 594\ \text{nm})$$

$$(4a)\quad \text{WB} + \text{NaOH} \xrightarrow{\text{pH 13}} \text{alkaline hematin} \xrightarrow{\text{DMLAO}} \text{GP-1}$$

$$(4b)\quad \text{WB} + \text{Borate Buffer} \xrightarrow{\text{pH 11.3}} \text{oxy Hb (FeII) DMLAO GP-1}$$

$$(4c)\quad \text{WB} + \text{Phosphate Buffer} \xrightarrow{\text{pH 7.5}} \text{Yellow Brown Product}$$

In Equation (1), whole blood (WB) which contains oxyhemoglobln (FeII), combines with 3% N,N-dimethylaurylamine N-oxide (DMLAO), which is a zwitterionic surfactant, in 0.5 N sodium hydroxide (NaOH) to yield a green end-product (GP-1) . Product GP-1 has an absorption spectrum having a maximum absorption at 570 nm, and a shoulder at 595 nm. When 3% sodium lauryl sulfate (SLS), an anionic surfactant, in 0.5N NaOH is reacted with whole blood (Equation 2), a product (GP-2) is formed, having an absorption spectrum with maxima at 495 nm and 603 nm and shoulder at 525 nm. When 3% cetyltrimethylammonium bromide (CTAB), a cationic surfactant, in 0.5 N NaOH is reacted with the whole blood (Equation 3), product (GP-3) results, with an absorption spectrum having a maximum at 594 nm.

Equations 1, 2 and 3 illustrate the effects of the surfactant head group charge on the spectrum of the reaction product. The resulting chromogens are now dispersed in surfactant micelles. The spectra of the resulting chromogens, as given above, indicate that the charge of the surfactant head group perturbs the electron configuration of the chromogen and the variations in electrostatic charge of the surfactant head group produce different absorption spectra. In these reactions, the rate of transformation is essentially instantaneous.

The DMLAO/NaOH reagent system (Equation 1) is more completely described by Equations 4a through 4c. In Equation 4a, the blood sample, when diluted with 0.5 N NaOH, rapidly yields red-brown alkaline hematin. However, if the blood sample is diluted initially at pH 11.3 by 50 mM borate, instead of 0.5 N NaOH, the spectrum of the oxyhemoglobin (having maxima at 540 and 576 nm) was retained. When DMLAO is added to oxyhemoglobin at pH 11.3, GP-1 was formed (Equation 4b). In contrast, when the blood sample was diluted by 50 mM phosphate at (pH 7.5), again oxyhemoglobin spectrum is observed and, after the addition of DMLAO, a yellow-brown product is formed (Equation 4c). Equations 4a and 4b show that the pH of the reaction is important and must be highly alkaline to promote the rapid transformation of oxyhemoglobin to GP-1. When a strong base is used in the absence of surfactant, alkaline hematin is shown to be a precursor to GP-1.

Since alkaline hematin is a ferric heme derivative (Merck Index, 9th Edition), the overall transformation of hemoglobin in blood to produce the absorption spectrum of GP-1 (Equation 1) necessarily involves oxidation of the heme iron ion from the ferrous state (FeII) to the ferric state (Fe III).

Other surfactants tested appear to serve a role similar to that of DMLAO. Lowering of the polarity of the reaction mixture serves to release the hydrophobic heme, which is non-covalently complexed with the globin and also is a causal factor for the color (absorption spectrum) of GP-1. In addition, the surfactant causes lysis of the red blood cells and fully disperses the resulting cell ghosts and emulsifies lipids present in the sample, so as to yield a reaction mixture free of turbidity. An advantage of the production of the green end-product, GP-1, is that detection at 570 nm avoids bilirubin interference. Also, GP-1 is generated very rapidly, i.e., within 5 s after mixing blood and reagent, and is stable for several hours, thus suitable for manual determination of hemoglobin concentration.

Accordingly, a characteristic absorption spectrum given by GP-1 indicative of hemoglobin concentration of the blood sample is achieved using a reagent composition which is completely cyanide-free, which provides distinct advantages in reagent manufacture, transportation, use in the laboratory and waste

disposal.

EXAMPLE I

Linearity and Correlation at pH of 13.7

This example illustrates that a linear relationship exists between the total hemoglobin concentration and the resulting absorbence, at a specified wavelength, of the blood sample reacted with the reagent composition of the present invention. Linear behavior, indicative of adherence to Beer's Law, is a desirable property of any colorimetric assay and facilitates standardization and calibration of the assay. Acceptable precision in the field of hemoglobinometry is characterized by a coefficient of variation of $\pm$ 1.0%.

The reagent composition used in performance of the method was prepared as follows. NaOH (20 g) was dissolved in 800 ml of deionized water. Thereafter, 133.4 ml of 30% aqueous N,N-dimethylaurylamine N-oxide was added, with gentle mixing. The preparation was brought up to 1 l with deionized water and filtered to remove particulates (0.2 µm filter). The resulting solution consisted of 0.5 N NaOH and 4% DMLAO and had a pH of 13.7.

Ten tubes of whole blood (10 ml) were collected in standard EDTA Vacutainers (Becton Dickenson Vacutainer Systems, Rutherford, N.J.) and pooled. The cell/plasma ratio of the pooled blood was manipulated to provide a series of samples which varied with respect to total hemoglobin concentration. By "manipulated" is meant to vary in controlled fashion the original hemoglobin concentration, i.e., hematocrit. The tubes of whole blood were centrifuged (5 min x 3000 rpmin) to separate the red blood cells from the plasma and buffy coat in each. The buffy coat was discarded and the packed red blood cells were resuspended by volumetric pipetting, in varying proportions, in their native plasma. Six levels of hemoglobin were generated to cover the clinical range between 2.5-25 g/dl. Each level was assayed in (5) replicates, by the mixing of 2 µl of the sample with 500 µl of reagent (1:250), prepared as described above. The resulting mixture was then passed through a flow cell (path length = 0.80 cm) of a colorimeter equipped with a 570 $\pm$ 4 nm band width filter. Absorbence values were taken 25 after mixing sample and reagent. The results are presented in Table I.

TABLE I

| Sample | Hematocrit | $A_{570}$ ±CV% |
|---|---|---|
| 1 | 0 | 0.000 ± 0 |
| 2 | 15 | 0.083 ± 0.54 |
| 3 | 30 | 0.166 ± 0.29 |
| 4 | 45 | 0.251 ± 0.57 |
| 5 | 60 | 0.333 ± 0.56 |
| 6 | 75 | 0.412 ± 0.29 |

Table I indicates that the relationship between relative hemoglobin concentrations and measurement at $A_{570}$ is linear with a slope of 1.00 over the clinical range. Also, the calibration curve passes through the origin since absorbence is zero, i.e., $A_{570}$ = 0 when hemoglobin concentration is zero. Furthermore, the precision of the method, as shown by calculation of the coefficient of variation is less than or equal to 0.57%, which is well within accepted levels of precision for hemoglobinometry.

EXAMPLE II

Correlation with reference to Cyanmethemoglobin Method

This example indicates that the results of the present method correlate with those of the cyanmethemoglobin reference method.

Twenty whole blood samples were analyzed for their hemoglobin concentration by the method described in Example I and also by the cyanmethemoglobin reference method using cyanide (CN), described in Van kampen and Ziljstrs. See Table II which was adapted to the TECHNICON H-6000 Flow Cytometry System (Technicon Instruments Corporation, Tarrytown, NY) and operated in accordance with the manufacturer's protocol.

The correlation study yielded the following linear regression equation: $(Hb, g/dl)_{CN\text{-}Free} = 0.99\ (Hb, g/dl)_{REFERENCE} + 0.046$ g/dl

Thus, the method of the invention recovered 99% of the value obtained by the reference method.

TABLE II

Correlation of CN-Free Hb Method to the Reference Hb Method

| Sample | Hb,g/dl, CN-Free | Hb,g/dl, Reference |
|---|---|---|
| 1 | 11.5 | 11.9 |
| 2 | 12.3 | 12.5 |
| 3 | 14.5 | 14.5 |
| 4 | 8.10 | 8.25 |
| 5 | 11.1 | 11.2 |
| 6 | 11.8 | 11.8 |
| 7 | 16.3 | 16.4 |
| 8 | 8.40 | 8.40 |
| 9 | 16.2 | 16.4 |
| 10 | 10.6 | 10.7 |
| 11 | 11.8 | 11.8 |
| 12 | 11.7 | 12.0 |
| 13 | 9.30 | 9.35 |
| 14 | 8.90 | 8.95 |
| 15 | 14.6 | 14.6 |
| 16 | 11.4 | 11.5 |
| 17 | 7.90 | 7.80 |
| 18 | 8.90 | 8.80 |
| 19 | 10.4 | 10.4 |
| 20 | 14.6 | 14.7 |

In summary, the observed linearity, precision and accuracy of the present method are all well within the acceptable ranges required by clinical hemoglobinometry.

EXAMPLE III

Assay of Blood Poisoned with Carbon Monoxide

The hemoglobin in blood of certain individuals may be "poisoned" so as to contain carboxyhemoglobin due to contact with carbon monoxide. For example, the carboxyhemoglobin in blood samples of heavy smokers and taxi drivers may constitute up to 10% of the total hemoglobin content. An important aspect of any reliable method for hemoglobin analysis is the ability to provide for "poisoned" hemoglobin, the same end product as native hemoglobin, since the spectra of "poisoned" forms of hemoglobin would differ from that of the native hemoglobin.

Two tubes of blood were drawn. One tube was labelled "control". The second tube was labelled "carboxyhemoglobin". The second tube was placed in a hood and a stream of carbon monoxide was bubbled through the contained blood for 1 h. The effect of carbon monoxide was observed, the blood acquiring a distinct cherry-red color. The carboxyhemoglobin tube was then found to contain 100% carboxyhemoglobin.

The "control" and "carboxyhemoglobin" tubes were next assayed for hemoglobin by the method of Example I. The reaction products obtained in both tubes were substantially identical, each yielding the characteristic green end product GP-1 with a spectral maximum at 570 nm of substantially the same absorbence, as determined by a rapid scanning spectrophotometer. This supports the mechanism in which the heme is released from the interior of "poisoned" hemoglobin and enters into the reaction, since the same end product is produced, regardless of whether all or a portion of the hemoglobin is "poisoned". Accordingly, the method of the present invention is effective to assay for carboxyhemoglobin, either singly or in the presence of native hemoglobin, as indicated in Table III.

TABLE III

Response of "Control" and "Carboxyhemoglobin" Blood

| Blood | % COHb | $A_{570}$± CV,% | Hb,g/dl |
|---|---|---|---|
| "Control" | 0 | 0.251±0.57 | 15.2 |
| "Carboxyhemoglobin | 100±2 | 0.253±0.58 | 15.2 |

Both samples were assayed in triplicate to determine % COHb by the method described in N. Tietz, Fundamentals of Clinical Chemistry, 1970, p. 836, W. B. Saunders Company.

EXAMPLE IV

Linearity and Correlation at pH of 11.3

This Example illustrates the linearity and correlation of the present method using a cyanide-free reagent composition having a pH of 11.3. In Example I, above, the reagent composition has a pH of 13.7.

1 l of reagent was prepared, which comprised 19.06g of sodium borate, 66.7 ml of N,N-dimethyl laurylamine N-oxide (DMLAO), (30% stock solution in water), 4.0g of NaOH and sufficient additional NaOH to adjust the pH to 11.3 ± 0.2. The reagent was filtered (0.2µm) to remove any particulates. The final concentrations of compounds were: 2% DMLAO in 50 nM sodium borate buffer, pH 11.3.

This reagent and aliquots of the six manipulated blood samples as obtained in Example 1, were reacted in the manner described in Example I, except that absorbence of the reacted sample was measured 15 s after mixing. When plotted against hematocrit, the absorbence at 570 nm of the green end-product yields a straight line for hemoglobin concentrations in the range of 2.5-25 g/dl, as shown in Table IV. The correlation study vs. the reference method (described in Example II) yielded the following linear regression equation:

$(\text{Hb, g/dl})_{\text{CN-Free}} = 1.00\ (\text{Hb, g/dl})_{\text{REFERENCE}} + 0.22$ g/dl

## TABLE IV
## Linearity and Correlation of Cyanide-Free Hb Method at pH 11.3

| | | CN-Free Method | | Reference Method |
|---|---|---|---|---|
| Sample | Hct | $A_{570\ nm}$ | Hb,g/dl | Hb,g/dl |
| 1 | 0 | 0.000 | 0 | 0 |
| 2 | 15 | 0.100 | 5.48 | 5.20 |
| 3 | 30 | 0.201 | 10.8 | 10.4 |
| 4 | 45 | 0.303 | 16.0 | 15.7 |
| 5 | 60 | 0.401 | 21.1 | 20.7 |
| 6 | 75 | 0.503 | 25.9 | 26.0 |

The precision for the CN-free Hb method (5 replicates per Hb level) was 0.57%.

## Claims

1. A cyanide-free aqueous solution for determining the total hemoglobins concentration in a blood sample comprising an ionic surfactant having a pH of at least 11.3, said surfactant containing an alkyl hydrophobic member containing up to 30 carbon atoms including a principal chain containing from 10 to 20 carbon atoms and a polar group which is zwitterionic, cationic or anionic.

2. The aqueous solution of claim 1 wherein said alkyl hydrophobic member contains up to 24 carbon atoms including a principal chain containing 12 to 18 carbon atoms.

3. The aqueous solution of claim 1 or 2 wherein said ionic surfactant is a cationic surfactant which is not basic enough to impart the required pH by itself.

4. The aqueous solution of claim 3 wherein said cationic surfactant has the structural formula

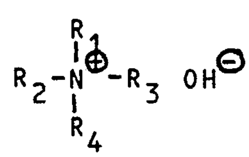

wherein $R_1$ is $C_{12}$-$C_{18}$ alkyl; and $R_2$, $R_3$ and $R_4$ are the same or different and are lower alkyl wherein the total number of carbon atoms of the alkyl groups of $R_2$, $R_3$ and $R_4$ when added to the number of carbon atoms of the alkyl group of $R_1$ does not exceed 24.

5. The aqueous solution of claim 4 wherein said composition has a pH of at least 13.7 and said cationic surfactant is a $C_{12}$-$C_{18}$ alkyltrimethylammonium hydroxide.

6. The aqueous solution of claim 5 wherein said $C_{12}$-$C_{18}$ alkyltrimethylammonium hydroxide is selected from the group consisting of stearyltrimethylammonium hydroxide, lauryltrimethylammonium hydroxide and myristyltrimethylammonium hydroxide.

7. The aqueous solution of claim 3 wherein said composition includes a strong base and said cationic surfactant has the structural formula

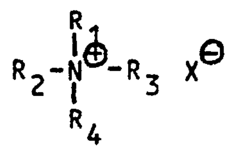

wherein $R_1$ is $C_{12}$-$C_{18}$ alkyl; $R_2$, $R_3$ and $R_4$ are the same or different and are hydrogen or lower alkyl wherein the total number of carbon atoms of the alkyl groups of $R_2$, $R_3$ and $R_4$ when added to the number of carbon atoms of the alkyl group of $R_1$ does not exceed 24; and X is a halogen.

8. The aqueous solution of claim 7 wherein said composition has a pH of at least 13.7 and said cationic surfactant is a $C_{12}$-$C_{18}$ alkyltrimethylammonium halide.

9. The aqueous solution of claim 2 wherein said ionic surfactant is a zwitterionic surfactant.

10. The aqueous solution of claim 9 wherein said zwitterionic surfactant is a N-oxide of a $C_{12}$-$C_{18}$ alkylamine having the structural formula

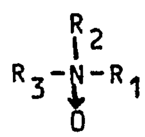

wherein $R_1$ is $C_{12}$-$C_{18}$ alkyl; and $R_2$ and $R_3$ are the same or different and are lower alkyl wherein the total number of carbon atoms of the alkyl groups of $R_2$ and $R_3$ when added to the number of carbon atoms of the alkyl group of $R_1$ does not exceed 24.

11. The aqueous solution of claim 10 wherein said composition has a pH of at least 13.7 and said zwitterionic surfactant is a N,N-dimethyl-$C_{12}$-$C_{18}$ alkylamine N-oxide.

12. The aqueous solution of claim 9 wherein said composition includes an independent base compound and said N,N-dimethyl $C_{12}$-$C_{18}$ alkylamine N-oxide is selected from the group consisting of N,N-dimethyllaurylamine N-oxide, N,N-dimethylcetylamine N-oxide and N,Ndimethylstearylamine N-oxide.

13. The aqueous solution of claim 2 wherein said ionic surfactant is anionic.

14. The aqueous solution of claim 13 wherein said anionic surfactant is a metal salt of a $C_{12}$-$C_{18}$ alkyl sulfate wherein said metal is selected from the group consisting of alkali metals and alkaline earth metals.

15. The aqueous solution of claim 14 wherein said composition has a pH of at least 13.7 and includes a strong hydroxide base.

16. The aqeous solution of claim 15 wherein said metal salt of a $C_{12}$-$C_{18}$ alkyl sulfate is selected from the group consisting of sodium lauryl sulfate, lithium lauryl sulfate and sodium myristyl sulfate and said

hydroxide base is selected from the group consisting of sodium hydroxide, potassium hydroxide and a tetra-$C_1$-$C_4$ alkylammonium hydroxide.

**17.** A method for determining the total concentration of hemoglobins in a blood sample which comprises:
(a) treating red blood cells in a blood sample with the aqueous solution of claim 1 wherein an oxidized heme product is formed; and
(b) measuring the absorbance of said oxidized heme product and calculating the hemoglobins concentration therefrom.

**18.** The method of claim 17 wherein the ionic surfactant of the aqueous solution of claim 1 is a zwitterionic surfactant and said measurement of absorbance of said oxidized heme product occurs at 570 nm.

**19.** The method of claim 17 wherein the ionic surfactant of the aqueous solution of claim 1 is a cationic surfactant and said measurement of absorbance of said oxidized heme product occurs at 594 nm.

**20.** The method of claim 17 wherein the ionic surfactant of the aqueous solution of claim 1 is an anionic surfactant and said measurement of absorbance of said oxidized heme product occurs at 594 nm.

## Revendications

**1.** Solution aqueuse exempte de cyanure pour la détermination de la concentration totale en hémoglobines dans un échantillon de sang, comprenant un agent tensio-actif ionique ayant un pH d'au moins 11,3, ledit agent tensio-actif contenant un élément hydrophobe de type alkyle contenant jusqu'à 30 atomes de carbone, incluant une chaîne principale contenant 10 à 20 atomes de carbone, et un groupe polaire qui est zwitterionique, cationique ou anionique.

**2.** Solution aqueuse selon la revendication 1, dans laquelle ledit élément hydrophobe de type alkyle contient jusqu'à 24 atomes de carbone incluant une chaîne principale contenant 12 à 18 atomes de carbone.

**3.** Solution aqueuse selon la revendication 1 ou 2, dans laquelle ledit agent tensio-actif ionique est un agent tensio-actif cationique qui n'est pas suffisamment basique pour conférer par lui-même le pH requis.

**4.** Solution aqueuse selon la revendication 3, dans laquelle ledit agent tensio-actif cationique a la formule structurale:

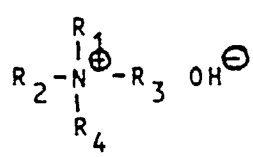

dans laquelle:
- $R_1$ représente alkyle en $c_{12}$-$c_{18}$; et
- $R_2$, $R_3$ et $R_4$ sont identiques ou différents et représentent alkyle inférieur, le nombre total d'atomes de carbone des groupes alkyle de $R_2$, $R_3$ et $R_4$, lorsqu'il est ajouté au nombre d'atomes de carbone du groupe alkyle de $R_1$, ne dépassant pas 24.

**5.** Solution aqueuse selon la revendication 4, dans laquelle ladite composition a un pH d'au moins 13,7 et ledit agent tensio-actif cationique est l'hydroxyde d'(alkyl en $C_{12}$-$C_{18}$>triméthylammonium.

**6.** Solution aqueuse selon la revendication 5, dans laquelle ledit hydroxyde d'(alkyl en $c_{12}$-$c_{18}$) triméthylammonium est choisi dans le groupe constitué par l'hydroxyde de stéaryltriméthylammonium, l'hydroxyde de lauryltriméthylammonium et l'hydroxyde de myristyltriméthylammonium.

**7.** Solution aqueuse selon la revendication 3, dans laquelle ladite composition comprend une base forte et ledit agent tensio -actif cationique a la formule structurale:

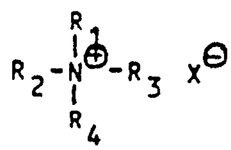

dans laquelle:
- $R_1$ représente alkyle en $C_{12}$-$C_{18}$;
- $R_2$, $R_3$ et $R_4$ sont identiques ou différents et représentent hydrogène ou alkyle inférieur, le nombre total d'atomes de carbone des groupes alkyle de $R_1$, $R_2$ et $R_3$, lorsqu'il est ajouté au nombre d'atomes de carbone du groupe alkyle de $R_1$, ne dépassant pas 24; et
- X représente un halogène.

**8.** Solution aqueuse selon la revendication 7, dans laquelle ladite composition a un pH d'au moins 13,7 et ledit agent tensio-actif cationique est un halogénure d'(alkyl en $C_{12}$-$C_{18}$)triméthylammonium.

**9.** Solution aqueuse selon la revendication 2, dans laquelle ledit agent tensio-actif ionique est un agent tensio-actif zwitterionique.

**10.** Solution aqueuse selon la revendication 9, dans laquelle ledit agent tensio-actif zwitterionique est un N-oxyde d'une (alkyl en $C_{12}$-$C_{18}$)amine ayant la formule structurale:

3

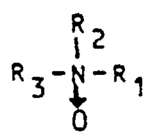

dans laquelle:
- $R_1$ représente alkyle en $C_{12}$-$C_{18}$; et
- $R_2$ et $R_3$ sont identiques ou différents et représentent alkyle inférieur, le nombre total d'atomes de carbone des groupes alkyle de $R_2$ et $R_3$, lorsqu'il est ajouté au nombre d'atomes de carbone du groupe alkyle de $R_1$, ne dépassant pas 24.

**11.** Solution aqueuse selon la revendication 10, dans laquelle ladite composition a un pH d'au moins 13,7 et ledit agent tensio-actif zwitterionique est un N-oxyde de N,N-diméthyl-(alkyl en $C_{12}$-$C_{18}$)-amine.

**12.** Solution aqueuse selon la revendication 9, dans laquelle ladite composition comprend un composé de type base indépendant et ledit N-oxyde de N,N-diméthyl-(alkyl en $C_{12}$-$C_{18}$)-amine est choisi dans le groupe constitué par le N-oxyde de N,N-diméthyl-laurylamine, le N-oxyde de N,N-diméthyl-cétylamine et le N-oxyde de N,N-diméthyl-stéarylamine.

**13.** Solution aqueuse selon la revendication 2, dans laquelle ledit agent tensio-actif ionique est anionique.

**14.** Solution aqueuse selon la revendication 13, dans laquelle ledit agent tensio-actif anionique est un sel métallique d'un sulfate d'alkyle en $C_{12}$-$C_{18}$, dans lequel ledit métal est choisi dans le groupe constitué par les métaux alcalins et les métaux acalino-terreux.

**15.** Solution aqueuse selon la revendication 14, dans laquelle la dite composition a un pH d'au moins 13,7 et comprend une base forte de type hydroxyde.

16. Solution aqueuse selon la revendication 15, dans laquelle ledit sel métallique d'un sulfate d'alkyle en $C_{12}$-$C_{18}$ est choisi dans le groupe constitué par le lauryl sulfate de sodium, le lauryl sulfate de lithium et le myristyl sulfate de sodium, et ladite base de type hydroxyde est choisie dans le groupe constitué par l'hydroxyde de sodium, l'hydroxyde de potassium et un hydroxyde de tétra-(alkyl en $C_1$-$C_4$)-ammonium.

17. Procédé de détermination de la concentration totale en hémoglobines dans un échantillon de sang, qui comprend :
   (a) le traitement des globules rouges d'un échantillon de sang par la solution aqueuse de la revendication 1, dans laquelle un produit de type hème oxydé est formé; et
   (b) la mesure de l'absorbance dudit produit de type hème oxydé et le calcul à partir de celle-ci de la concentration en hémoglobines.

18. Procédé selon la revendication 17, dans lequel l'agent tensio-actif ionique de la solution aqueuse de la revendication 1 est un agent tensio-actif zwitterionique, et ladite mesure d'absorbance dudit produit de type hème oxydé a lieu à 570 nm.

19. Procédé selon la revendication 17, dans lequel l'agent tensio-actif ionique de la solution aqueuse de la revendication 1 est un agent tensio-actif cationique et ladite mesure d'absorbance dudit produit de type hème oxydé a lieu à 594 nm.

20. Procédé selon la revendication 17, dans lequel l'agent tensio-actif ionique de la solution aqueuse de la revendication 1 est un agent tensio-actif anionique, et ladite mesure d'absorbance dudit produit de type hème oxydé a lieu à 594 nm.

## Ansprüche

1. Cyanidfreie wäßrige Lösung zur Bestimmung der Gesamtkonzentration an Hämoglobinen in einer Blutprobe, umfassend ein ionisches oberflächenaktives Mittel mit einem pH von mindestens 11,3, wobei das oberflächenatkive Mittel einen hydrophoben Alkyl-Teil enthält, der bis zu 30 Kohlenstoffatome enthält, beinhaltend eine Hauptkette mit 10 bis 20 Kohlenstoffatomen und eine polare Gruppe, die zwitterionisch, kationisch oder anionisch ist.

2. Wäßrige Lösung nach Anspruch 1, wobei der hydrophobe Alkylteil bis zu 24 Kohlenstoffatome enthält, beinhaltend eine Hauptkette mit 12 bis 18 Kohlenstoffatomen.

3. Wäßrige Lösung nach Anspruch 1 oder 2, wobei das ionische oberflächenaktive Mittel ein kationisches oberflächenatkives Mittel ist, das nicht ausreichend basisch ist, um den erforderlichen pH selbst vorzusehen.

4. Wäßrige Lösung nach Anspruch 3, wobei das kationische oberflächenaktive Mittel die Strukturformel

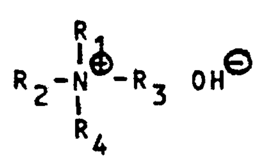

besitzt, worin bedeuten:
   $R_1$ $C_{12}$-$C_{18}$-Alkyl; und
   $R_2$, $R_3$ und $R_4$, die gleich oder verschieden sind, Niederalkyl, worin die Gesamtanzahl der Kohlenstoffatome in den Alkylgruppen von $R_2$, $R_3$ und $R_4$, wenn sie zur Anzahl der Kohlenstoffatome der Alkylgruppe von $R_1$ addiert werden, 24 nicht überschreitet.

5. Wäßrige Lösung nach Anspruch 4, wobei die Zusammensetzung ein pH von mindestens 13,7 besitzt und das kationische oberflächenaktive Mittel ein $C_{12}$-$C_{18}$-Alkyltrimethylammoniumhydroxid ist.

6. Wäßrige Lösung nach Anspruch 5, wobei das $C_{12}$-$C_{18}$-Alkyltrimethylammoniumhydroxid gewählt wird aus der Gruppe, bestehend aus Stearyltrimethylammoniumhydroxid, Lauryltrimethylammoniumhydroxid und Myristyltrimethylammoniumhydroxid.

7. Wäßrige Lösung nach Anspruch 3, wobei die Zusammensetzung eine starke Base beinhaltet und das kationische oberflächenaktive Mittel die Strukturformel

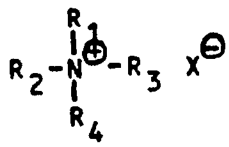

besitzt, worin bedeuten:
$R_1$ $C_{12}$-$C_{18}$-Alkyl;
$R_2$, $R_3$ und $R_4$, die gleich oder verschieden sind, ein Wasserstoff oder Niederalkyl, wobei die Gesamtanzahl der Kohlenstoffatome der Alkylgruppen von $R_2$, $R_3$ und $R_4$, wenn sie zur Anzahl der Kohlenstoffatome der Alkylgruppe von $R_1$ addiert werden, 24 nicht überschreitet; und
X ein Halogen.

8. Wäßrige Lösung nach Anspruch 7, wobei die Zusammensetzung einen pH von mindestens 13,7 besitzt und das kationische oberflächenaktive Mittel ein $C_{12}$-$C_{18}$-Alkyltrimethylammoniumhalogenid ist.

9. Wäßrige Lösung nach Anspruch 2, wobei das ionische oberflächenaktive Mittel ein zwitterionisches oberflächenaktives Mittel ist.

10. Wäßrige Lösung nach Anspruch 9, wobei das zwitterionische oberflächenaktive Mittel ein N-Oxid eines $C_{12}$-$C_{18}$-Alkylamins ist mit der Strukturformel

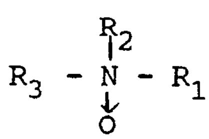

worin bedeuten:
$R_1$ $C_{12}$-$C_{18}$-Alkyl; und
$R_2$ und $R_3$ die gleich oder verschieden sind, Niederalkyl, worin die Gesamtanzahl der Kohlenstoffatome der Alkylgruppen von $R_2$ und $R_3$, wenn sie zu der Anzahl der Kohlenstoffatome der Alkylgruppe von $R_1$ addiert werden, 24 nicht überschreitet.

11. Wäßrige Lösung nach Anspruch 10, wobei die Zusammensetzung einen pH von mindestens 13,7 besitzt und das zwitterionische oberflächenaktive Mittel ein N, N-Dimethyl-$C_{12}$-$C_{18}$-alkylamin-N-Oxid ist.

12. Wäßrige Lösung nach Anspruch 9, wobei die Zusammensetzung eine unabhängige Basenverbindung beinhaltet und das N,N-Dimethyl-$C_{12}$-$C_{18}$-alkylamin-N-Oxid gewählt wird aus der Gruppe, bestehend aus N,N-Dimethyllaurylamin-N-Oxid, N,N-Dimethylcetylamin-N-Oxid und N,N-Dimethylstearylamin-N-Oxid.

13. Wäßrige Lösung nach Anspruch 2, wobei das ionische oberflächenaktive Mittel anionisch ist.

14. Wäßrige Lösung nach Anspruch 13, wobei das anionische oberflächenaktive Mittel ein Metallsalz eines $C_{12}$-$C_{18}$-Alkylsulfats ist, wobei das Metall gewählt wird aus der Gruppe, bestehend aus Alkalimetallen und Erdalkalimetallen.

15. Wäßrige Lösung nach Anspruch 14, wobei die Zusammensetzung einen pH von mindestens 13,7 besitzt und eine starke Hydroxid-Base beinhaltet.

**16.** Wäßrige Lösung nach Anspruch 15, wobei das Metallsalz eines $C_{12}$-$C_{18}$-Alkylsulfats gewählt wird aus der Gruppe, bestehend aus Natriumlaurylsulfat, Lithiumlaurylsulfat und Natriummyristylsulfat und die Hydroxid-Base gewählt wird aus der Gruppe, bestehend aus Natriumhydroxid, Kaliumhydroxid und einem Tetra-$C_1$-$C_4$-alkylammoniumhydroxid.

**17.** Verfahren zur Bestimmung der Gesamtkonzentration an Hämoglobinen in einer Blutprobe, umfassend:
(a) Behandeln roter Blutzellen in einer Blutprobe mit der wäßrigen Lösung nach Anspruch 1, worin ein oxidiertes Häme-Produkt gebildet wird; und
(b) Messen der Extinktion des oxidierten Häme-Produkts und Berechnen der Konzentration an Hämoglobinen daraus.

**18.** Verfahren nach Anspruch 17, wobei das ionische oberflächenaktive Mittel der wäßrigen Lösung nach Anspruch 1 eine zwitterionisches oberflächenaktives Mittel ist und die Messung der Extinktion des oxidierten Häme-Produkts bei 570 nm auftritt.

**19.** Verfahren nach Anspruch 17, wobei das ionische oberflächenaktive Mittel der wäßrigen Lösung nach Anspruch 1 ein kationisches oberflächenaktives Mittel ist und die Messung der Extinktion des oxidierten Häme-Produkts bei 594 nm auftritt.

**20.** Verfahren nach Anspruch 17, wobei das anionische oberflächenaktive Mittel der wäßrigen Lösung nach Anspruch 1 ein anionisches oberflächenaktives Mittel ist und die Messung der Extinktion des oxidierten Häme-Produkts bei 594 nm auftritt.